(19) Europäisches Patentamt
European Patent Office
Office européen des brevets

(11) **EP 3 468 607 B1**

(12) **EUROPÄISCHE PATENTSCHRIFT**

(45) Veröffentlichungstag und Bekanntmachung des Hinweises auf die Patenterteilung:
**02.12.2020 Patentblatt 2020/49**

(21) Anmeldenummer: **17729837.9**

(22) Anmeldetag: **06.06.2017**

(51) Int Cl.:
*A61K 31/05* (2006.01)    *A61K 47/14* (2017.01)
*A61K 47/26* (2006.01)    *A61K 9/10* (2006.01)
*A61K 9/107* (2006.01)    *A61K 9/48* (2006.01)

(86) Internationale Anmeldenummer:
**PCT/EP2017/063673**

(87) Internationale Veröffentlichungsnummer:
**WO 2017/215980 (21.12.2017 Gazette 2017/51)**

(54) **RESVERATROLSOLUBILISAT**

RESVERATROL SOLUBILITY

SOLUBILISAT DE RESVÉRATROL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.06.2016 PCT/EP2016/063577**

(43) Veröffentlichungstag der Anmeldung:
**17.04.2019 Patentblatt 2019/16**

(73) Patentinhaber: **Aquanova AG**
**64295 Darmstadt (DE)**

(72) Erfinder: **BEHNAM, Dariush**
**64380 Roßdorf (DE)**

(74) Vertreter: **Tesch, Sabine et al**
**Augspurger Tesch Friderichs**
**Patent- und Rechtsanwälte PartG mbB**
**Kaiserstraße 39**
**55116 Mainz (DE)**

(56) Entgegenhaltungen:
**WO-A2-2007/006497     DE-U1-202009 016 292**
**DE-U1-202012 012 130     KR-A- 20090 132 357**

EP 3 468 607 B1

**Beschreibung**

Die Erfindung betrifft ein Resveratrolsolubilisat.

Resveratrol ist ein Phytoalexin mit antioxidativen Eigenschaften, das zu den Polyphenolen zählt. Die Substanz findet sich beispielsweise in Weintrauben, in relativ großen Mengen dabei in der Haut von roten Weintrauben, jedoch auch in Himbeeren, Maulbeeren, Pflaumen, Erdnüssen und im Japanischen Staudenknöterich. Auch aus der Weinrebe selbst lässt sich Resveratrol isolieren. Gemäß dem Eintrag in der Online-Enzyklopädie "Wikipedia" haben In-vitro-Studien Hinweise auf eine mögliche Wirksamkeit gegen Krebszellen und positive Effekte bei Krankheiten wie Arteriosklerose, Herzkrankheiten, Alzheimer-Krankheit, Arthritis und manchen Autoimmunkrankheiten erbracht.

Nach einem Bericht, den die "Pharmazeutische Zeitung online" in der Ausgabe 29/2007 publizierte, ist die antioxidative Wirkung von Resveratrol allerdings nicht nur für den Gefäßschutz von Bedeutung. Als besonders lipidreiches Organ leidet auch das Gehirn unter übermäßigem oxidativen Stress. Eine neuroprotektive Wirkung von Resveratrol, das die Blut-Hirn-Schranke überwindet, wurde danach in mehreren In-vivo-Studien an Ratten nachgewiesen. Neuere Arbeiten deuteten sogar darauf hin, dass Resveratrol direkt die Prozessierung von Beta-Amyloid, dem pathogenen Faktor der Alzheimer-Demenz, beschleunigt.

Neben der oxidativen Belastung wird die chronisch niederschwellige Entzündung als ein Faktor für beschleunigt ablaufende Alterungsprozesse diskutiert. Nach dem genannten Bericht führt der intrazelluläre Signalweg, der eine vermehrte Produktion proinflammatorischer Zytokine bewirkt, über den nukleären Faktor kappa-b (NFκ-b). Dieser kann durch viele Stimuli (UV-Strahlung, Bakterientoxine) aktiviert werden und wandert dann in den Zellkern, wo er die Genexpression unterschiedlicher Entzündungsenzyme induziert. NFκ-b wird zunehmend als die entscheidende Schaltstelle für die Verknüpfung oxidativer und inflammatorischer Prozesse verstanden. Resveratrol inhibiert in vitro die nukleäre Translokation von NFκ-b und unterbindet somit einen der wichtigsten Mechanismen in der Genese proinflammatorischer Mediatoren.

Eine der vielfältigen Wirkungen von Resveratrol ist hoch spezifisch für diese Substanz. Resveratrol hat auf unterschiedliche Organismen die gleiche lebensverlängernde Wirkung wie eine anhaltende Kalorienrestriktion (Calorie restriction, CR). Es zählt somit zu den CR-Mimetika.

Auch gemäß Nathan Gray "Resveratrol could enhance exercise performance" (20.06.2012, www.nutraingredients.com/content/view/print/648155) weisen weitere Studien an diversen Organismen auf die Wirkung von Resveratrol gegen Krebs und Diabetes, eine Schutzwirkung durch Resveratrol vor Alzheimer sowie auf die entzündungshemmenden Eigenschaften und positive kardiovaskuläre Effekte von Resveratrol hin.

Allerdings ist bei bekannten Formulierungen von Resveratrol problematisch, dass diese eine extrem geringe Absorption im Organismus des Patienten, beziehungsweise Bioverfügbarkeit aufweisen. Zur Erhöhung der Bioverfügbarkeit ist der Einsatz weiterer Komponenten neben Resveratrol zur Schaffung von Trägersystemen wie beispielsweise Emulsionen oder Liposomen bekannt. Während in Emulsionen das Resveratrol in einer lipophilen Phase gelöst und in Tropfenform in wässriger Umgebung stabilisiert wird, kann bei Liposomen das Resveratrol in einer Phospholipidschicht gehalten werden. So kann die Bioverfügbarkeit gegenüber der nativen Form zwar gesteigert werden, jedoch sind derartige Formulierungen wie Liposome mechanisch äußerst instabil und auch nicht resistent gegenüber dem Milieu im Magen.

Für Anwendungen in Nahrungsergänzungsmitteln und Getränken ist zudem nachteilig, dass die bekannten Formulierungen nicht transparent sind und keine wässrige klare Lösung ergeben.

Es ist eine Aufgabe der Erfindung, eine oral zu verabreichende Formulierung bereit zu stellen, die hinreichend stabil ist. Insbesondere ist es eine Aufgabe der Erfindung, eine Formulierung für den Wirkstoff Resveratrol zu schaffen, in der Resveratrol eine Bioverfügbarkeit hat, die eine Einnahme in gegenüber den erforderlichen aufzunehmenden Mengen von nativem Resveratrol deutlich verringerter Masse erlaubt. Die Optimierung der Absorption von Resveratrol durch eine entsprechend geeignete Formulierung ist dabei eine Aufgabe der Erfindung. Des Weiteren ist es eine Aufgabe der Erfindung, eine stabil homogene Feinverteilung von Resveratrol in den entsprechenden Endprodukten, wie Lebens- und Nahrungsergänzungsmitteln zu erreichen.

Die Erfindung stellt eine micellare Resveratrolformulierung gemäß den Ansprüchen zur Verfügung, auf deren Basis im Vergleich zu nativem Resveratrol eine deutlich höhere Bioverfügbarkeit festgestellt werden konnte. Die Erfindung stellt ein Solubilisat bestehend aus Resveratrol, einer Mischung aus Polysorbat 80 und Polysorbat 20 sowie zumindest einem mittelkettigen Triglycerid zur Verfügung.

Die erfindungsgemäße Formulierung schafft durch das Solubilisat mit Resveratrol beladene Micellen. Überraschenderweise hat sich herausgestellt, dass die Verwendung allein von Polysorbat 80 oder allein von Polysorbat 20 nicht zu den gewünschten stabilen Mizellen führt, welche auch bei den im Magen gegebenen extrem sauren Bedingungen stabil bleiben und so erst über die Dünndarmwand das Resveratrol an den Organismus abgeben. Erst durch den Einsatz beider Emulgatoren in Kombination mit zumindest einem mittelkettigen Triglycerid gelang es dem Erfinder, ein Solubilisat mit solch stabilen Mizellen zu schaffen.

Mittelkettige Triglyceride, engl. medium-chain triglycerides (MCTs) sind Triglyceride, die mittelkettige Fettsäuren enthalten. Zu den mittelkettigen Fettsäuren zählen die Capronsäure, Caprylsäure, Caprinsäure und die Laurinsäure. Es handelt sich dabei um gesättigte Fettsäuren, welche natürlich in tropischen Pflanzenfetten wie Kokosfett und Palmkernöl vorkommen. Zu einem geringen Teil sind sie auch im Milchfett enthalten. In der Natur gibt es kein reines MCT-Öl, synthetisch lassen sich jedoch reine MCT-Öle gewinnen. Im Rahmen der Erfindung können als mittelkettige Triglyceride einzelne MCTs oder eine Mischung unterschiedlicher MCTs eingesetzt werden.

Die Erfindung schafft die Möglichkeit, eine Resveratrolformulierung mit einer hohen Beladung der Micellen mit Resveratrol realisieren zu können, ohne dass die Micellen aufplatzen und das Resveratrol bei Verdünnung mit Wasser als Sediment freigesetzt wird.

Der Anteil an Resveratrol in dem erfindungsgemäßen Solubilisat kann im Rahmen der Erfindung bis hin zu sehr hohen Werten variiert werden, ohne die Mizellen zu destabilisieren. Der Resveratrolanteil liegt in einer bevorzugten Ausgetaltung des Solubilisats im Bereich zwischen 3 Gew.-% und 15 Gew.-%, besonders bevorzugt im Bereich zwischen 5 Gew.-% und 10 Gew.-%, und beträgt insbesondere 10 Gew.-%.

Die Emulgatormischung aus Polysorbat 20 und Polysorbat 80 liegt im erfindungsgemäßen Solubilisat in einer Menge im Bereich zwischen mindestens etwa 65 Gew.-% und etwa 95 Gew.-% vor, insbesondere in einer Menge im Bereich zwischen etwa 70 Gew.-% und etwa 92 Gew.-%, bevorzugt beträgt der Anteil der Emulgatormischung etwa 71,8 Gew.-%.

In einer vorteilhaften Weiterbildung liegt im Solubilisat das zumindest eine mittelkettiges Triglycerid in einer Menge im Bereich zwischen mindestens etwa 2 Gew.-% und etwa 8 Gew-% vor, insbesondere in einer Menge im Bereich zwischen etwa 3 Gew.-% und etwa 5 Gew.-%, wobei der Anteil des zumindest einen mittelkettigen Triglycerids (MCT-Anteil) bevorzugt etwa 4,5 Gew.-% beträgt.

Überraschenderweise hat sich herausgestellt, dass die Beladungsfähigkeit des Solubilisat gesteigert werden kann, indem dem Solubilisat als weiterer Bestandteil Tocopherol, insbesondere Mischtocopherol, zugegeben wird. Wird Tocopherol eingesetzt, hat es sich unerwartet als vorteilhaft ergeben, den Anteil des zumindest einen mittelkettigen Triglycerids zu erhöhen. Bei einer Verwendung von beispielsweise 7,5 Gew.-% Tocopherol in Form von Mischtocopherol führt eine Erhöhung des MCT-Anteils von 4,0 Gew.-% auf 4,5 Gew.-% zu einer überproportionalen Erhöhung der Beladungsfähigkeit mit Resveratrol von 5 Gew.-% auf 10 Gew.-%.

Im Rahmen der Erfindung kann der Tocopherolanteil variiert werden, wobei sich gezeigt hat, dass eine Menge im Bereich bis etwa 10 Gew.-% ausreichend ist. Insbesondere liegt der Tocopherolanteil in dem erfindungsgemäßen Solubilisat in einer Menge im Bereich zwischen etwa 3 Gew.-% und etwa 6 Gew.-% vor, und bevorzugt beträgt der Tocopherolanteil etwa 5,25 Gew.-%.

Je nach Anwendungsbereich kann im Rahmen der Erfindung das Solubilisat mit α-Tocopherol und/oder β-Tocopherol und/oder γ-Tocopherol und/oder δ-Tocopherol oder mit einem Mischtocopherol aus α-Tocopherol, β-Tocopherol, γ-Tocopherol und δ-Tocopherol hergestellt werden.

Gegenüber der Verwendung beispielsweise von α-Tocopherol alleine hat sich gezeigt, dass der Einsatz derselben Menge an Mischtocopherol ein größeres antioxidatives Potential im erfindungsgemäßen Solubilisat entfaltet.

Chemische lipophile Antioxidantien können im Rahmen der Erfindung zusätzlich oder alternativ zu Tocopherol auch eingesetzt werden. Beispielsweise kommen Butylhydroxyanisol (BHA; E320), Butylhydroxytoluol (BHT, E321), Gallate (E310 bis 312) oder Rosmarinextrakt mit den wirksamen Bestandteilen Carnosol und Carnosolsäure (E392) in Frage. Die angegebenen sogenannten "E-Nummern" beziehen sich dabei auf die Liste der von der Europäischen Union zugelassenen Lebensmittelzusatzstoffe.

Die besonders geringe Größe der Micellen im erfindungsgemäßen Solubilisat führt zu einem klaren und dauerhaft transparenten Produkt. Dazu trägt auch die enge Partikelgrößenverteilung bei, da die der Durchmesserverteilung der Micellen bei pH 7 und Raumtemperatur, das heißt bei einer Temperatur im Bereich von etwa 18°C bis etwa 22°C, lediglich von etwa 1 nm bis etwa 25 nm reicht. Insbesondere sind gemittelt etwa 69,45 Vol.-% +/- 0,55 Vol.-% der Partikeln größer als 3,22 nm +/-0,06 nm und gemittelt etwa 30,55 Vol.-% +/- 0,55 Vol.-% der Partikeln größer als 12,74 nm +/- 1,04 nm.

Bei pH 1 und einer Temperatur von 37°C reicht die Durchmesserverteilung der Micellen von etwa 2 nm bis etwa 900 nm reicht. Insbesondere sind gemittelt etwa 60,35 Vol.-% +/- 1,25 Vol.-% der Partikeln größer als 10,33 nm +/- 0,43 nm und gemittelt etwa 31,75 Vol.-% +/- 9,15 Vol.-% der Partikeln größer als 161,85 nm +/- 4,25 nm.

Durch die kleinen Partikelgrößen wird vorteilhafterweise die Ausbildung einer insbesondere für die Wahrnehmung mit dem menschlichen Auge klaren Flüssigkeit erreicht.

Die oben charakterisierten Partikelgrößenverteilungen der Micellen wurde gemessen nach dem Prinzip der dynamischen Lichtstreuung mit Laserlicht der Wellenlänge 780 nm. Die Partikelgrößenmessungen wurden mit dem ParticleMetrix NANOFLEX Rückstreu-Teilchenanalysator durchgeführt. Das Messprinzip beruht auf der dynamischen Lichtstreuung (DLS) in einer 180° Heterodyn-Rückstreuanordnung. Bei dieser Geometrie wird zum gestreuten Licht ein Teil des Laserstrahls dazu gemischt (Heterodyn-Technik). Wegen des geringen Lichtweges von 200 Mikrometer bis 300 Mikrometer in der Probe ist die Rückstreuung für absorbierende und hochkonzentrierte Proben von Vorteil. Die Heterodyn-Technik wirkt sich verstärkend auf das Signal/Rausch-Verhältnis und auf die Empfindlichkeit des Sub-100nm-Bereiches aus.

Das Laserlicht wird in die Y-Gabel einer Lichtfaser eingekoppelt. Zurück kommen in derselben Faser das am Saphirfenster der Probenkammer teilreflektierte Laserlicht und das von der Probe rückwärts gestreute Licht. Der Detektor im zweiten Ast der Y-Gabel nimmt die miteinander interferierenden Signale auf. Eine schnelle Fouriertransformations-Auswertung zerlegt die fluktuierenden Streulichtanteile in ein frequenzabhängiges sogenanntes "Power-Spektrum". Jeder Frequenzanteil stellt eine Brown'sche Diffusionskonstate dar und ist damit einer Partikelgröße zuzuordnen. Zur Umrechnung in eine Partikelgrößenverteilung wird die Stokes-Einstein-Formel verwendet:

$$D = k \frac{T}{3\pi\eta d_P}$$

In diese Gleichung gehen ein die Diffusionskonstante D, die Boltzmannkonstante k, die Temperatur T, die dynamische Viskosität $\eta$ des Mediums und der Durchmesser dp der Partikel. Ein Temperatursensor ist im Messgerät probennah in der Nähe des Saphirfensters angebracht.

Die Proben wurden je einmal im Verhältnis 1:10 mit vollentsalztem Wasser verdünnt. Das Solubilisat wurde dazu unter Rühren im Wasser gelöst. Es ist vollständig klar in Wasser löslich. Diese Lösung ist stabil und transparent. Anschließend wurde mit dem NANOFLEX je drei mal über einen Zeitraum von 30 Sekunden gemessen, aus den Messwerten wurde der Mittelwert gebildet.

Des Weiteren wurden die Proben auf einen pH-Wert von 1 eingestellt und bei 37°C unter ansonsten den gleichen Bedingungen vermessen. Damit wurden physiologische Bedingungen hinsichtlich der Magenpassage des Solubilisats nachgestellt.

Die Klarheit des Solubilisats läßt sich auch durch seine geringe Trübung darstellen.

Dazu wird folgende Arbeitshypothese angewandt: Je klarer eine wässrige Verdünnung eines Solubilisats oder einer anderen Formulierung von Resveratrol, insbesondere unter physiologischen Bedingungen, also bei einem pH-Wert von 1,1 und einer Temperatur von 37°C, ist, desto besser ist dessen Solubilisation. Je besser die Solubilisation, desto besser ist die Bioverfügbarkeit.

Diese lässt sich bereits an der besonders geringen Trübung des Solubilisats ablesen, welche sich als eine Art Kenngröße für die Bioverfügbarkeit verstehen lässt. Die Trübung des erfindungsgemäßen Solubilisats ist kleiner als 50 FNU gemessen durch Streulichtmessung mit Infrarotlicht nach den Vorschriften der Norm ISO 7027 bei einer Verdünnung des Solubilisats im Verhältnis 1:50 in Wasser.

Diese geringe Trübung behält das erfindungsgemäße Solubilisat auch nach 24 Stunden Lagerung bei 21°C und pH 7 ebenso wie nach 1 Stunde Lagerung bei 37°C und pH 1,1, also einerseits unter Lagerbedingungen bei Raumtemperatur in wässriger Verdünnung und andererseits bei der Passage des Magens. Daher liegt das Resveratrol im erfindungsgemäßen Solubilisat nach jetzigem Verständnis des Erfinders auch nach Passage des Magens noch in Form der stabilen, sehr kleinen Micellen vor und kann daher besonders gut im weiteren Verdauungstrakt aufgenommen werden.

Zur experimentellen Bestimmung der Trübung werden die Trübungsmessgeräte mit einer Standardsuspension kalibriert. Die Anzeige erfolgt somit nicht in Form der gemessenen Lichtintensität, sondern als Konzentration der Kalibriersuspension. Bei der Messung einer beliebigen Suspension bedeutet also die Anzeige, dass die betreffende Flüssigkeit die gleiche Lichtstreuung verursacht wie die Standardsuspension der angezeigten Konzentration. Der international festgelegte Trübungsstandard ist Formazin. Zu den geläufigsten Einheiten gehört die Angabe "FNU", das heißt "Formazine Nephelometric Units". Dies ist die beispielsweise in der Wasseraufbereitung verwendete Einheit für die Messung bei 90° gemäss den Vorschriften der Norm ISO 7072.

In einer vorteilhaften Weiterbildung enthält das erfindungsgemäße Solubilisat als weiteren Bestandteil zumindest ein anderes Polyphenol, insbesondere Quercetin und/oder Catechin, welches die Wirkung von Resveratrol je nach Anwendungsgebiet in synergistischer Weise unterstützen kann.

Die erfindungsgemäße transparente und vollständig stabil wasserlösliche Resveratrolformulierung weist, ohne die oben genannten Hilfsstoffe wie in Weich- und Hartgelatinekapseln, in gelatinefreien Kapseln (hart und/oder weich) und in Getränken oder flüssigen auf Wasser basierenden Endprodukten pH-unabhängig eine stabile Transparenz und darüber hinaus eine deutlich verbesserte Bioverfügbarkeit auf. Produkte mit derartiger Transparenz und Wasserlöslichkeit, aber auch insbesondere so hoher Bioverfügbarkeit der Resveratrolformulierung werden seitens der relevanten Industrie dringend für innovative Produkte als Kapselfüllung sowie als transparente Resveratrol-Getränke gesucht. Eine Resveratrolformulierung, die diesen Anforderungen gerecht wird, existiert bisher nach Kenntnis des Erfinders noch nicht.

Gegenüber der nativen Form von Resveratrol konnte die Erfindung die Bioverfügbarkeit mit Hilfe der spezifischen Formulierung deutlich erhöhen..
Die deutlich gesteigerte Bioverfügbarkeit des Resveratrols in dem erfindungsgemäßen Solubilisats gegenüber der nativen Form erlaubt eine Reduzierung der Menge an Resveratrol, die ein Nutzer täglich oral einzunehmen hat. So hat es sich beispielsweise herausgestellt, dass zum Erreichen der Wirkung einer Tagesdosis von 3500 mg nativem Resveratrol die Gabe von 200 mg Resveratrol in einem erfindungsgemäßen Solubilisat ausreicht.

Grundsätzlich kann das Solubilisat sowohl äußerlich in Anwendung an Haut, Nägeln und/oder Haaren als auch innerlich durch Aufnahme in den Körper verwendet werden. Dem Einsatz des Solubilisats stehen sämtliche Anwendungsformen offen, insbesondere die orale, dermale, intravenöse oder inhalative Verabreichung des Solubilisats oder eines Solubilisathaltigen Fluids.

Es hat sich vorteilhafterweise herausgestellt, dass das erfindungsgemäße Solubilisat in einfacher Form in Kapseln zur oralen Einnahme zur Verfügung gestellt werden kann, da es die Kaseln nicht angreift. Die Erfindung stellt damit auch eine Kapsel gefüllt mit dem Solubilisat zur Verfügung, wobei die Kapsel als Weichgelatinekapsel oder Hartgelatinekapsel oder als weiche, gelatinefreie Kapsel oder als harte, gelatinefreie Kapsel ausgebildet sein kann.

Eine weitere Darreichungsform ist ein Fluid enthaltend das erfindungsgemäße Solubilisat wobei das Fluid ein Lebensmittel, ein Getränk, ein kosmetisches Produkt wie insbesondere eine Creme, Lotion oder Salbe sein kann. Insbesondere kann das Fluid eine wässrige Verdünnung des Solubilisats umfassen. Die Anwendbarkeit des erfindungsgemäßen Solubilisats in einem Fluid ist nicht an dessen Viskosität gebunden, ebenso kann das Solubilisat sowohl in hydrophile als auch in lipophile Medien eingearbeitet werden.

Durch die mit Hilfe der Erfindung erreichbare gegenüber der nativen Form erhöhte Bioverfügbarkeit können die täglichen Dosen im Vergleich mit der oralen Gabe von nativem Resveratrol vorteilhafterweise deutlich verringert werden.

Im Folgenden werden Ausführungsbeispiele für Solubilisate gemäß der Erfindung erläutert.

Beispiel 1: Resveratrol-Solubilisat

Zur Herstellung eines Resveratrol-Solubilisats ohne Tocopherol wurden

| | |
|---|---|
| 50 g | Resveratrol, |
| 40 g | mittelkettige Triglyceride, |
| 840 g | Polysorat 80 und |
| 70 g | Polysorbat 20 |

verwendet.

Als Resveratrol wurde (trans-)Resveratrol, 99%ig mit dem Handelsnamen resVIDA der Koninklijke DSM N.V., Heerlen, Niederlande eingesetzt.

Mittelkettige Triglyceride wurden als MCT-Öl Delios VK koscher des Herstellers Cognis GmbH, Monheim, Deutschland, eingesetzt.

Als Polysorbat 80 wurde Crillet 4/Tween 80-LQ-(SG), Croda GmbH, Nettetal, Deutschland verwendet.

Als Polysorbat 20 wurde Crillet 1/Tween 20-LQ-(SG), Croda GmbH, Nettetal, Deutschland verwendet.

Polysorbat 20 und Polysorbat 80 wurden gemischt und auf eine Temperatur im Bereich zwischen etwa 50°C und etwa 70°C erwärmt.

In die Mischung aus Polysorbat 20 und Polysorbat 80, wurde das MCT-Öl bei einer Temperatur im Bereich zwischen etwa 50°C und etwa 70°C zugegeben und unter Rühren homogenisiert.

In die Mischung aus Polysorbat 20, Polysorbat 80, und MCT-Öl wurde das Resveratrol gegeben und zum Homogenisieren unter Rühren erhitzt auf eine Temperatur im Bereich zwischen etwa 83°C und etwa 87°C. Sobald das Fluid homogen und transparentes ist, wird es auf eine Temperatur unterhalb von etwa 60°C abgekühlt und abgefüllt.

Beispiel 2: Resveratrol-Solubilisat mit Mischtocopherol

Zur Herstellung des Solubilisats werden allein

| | |
|---|---|
| 100 g | Resveratrol, |
| 45 g | mittelkettige Triglyceride, |
| 600 g | Polysorat 80, |
| 180 g | Polysorbat 20 und |
| 75 g | Mischtocopherol |

verwendet.

Als Resveratrol wurde (trans-)Resveratrol, 99%ig bezogen von der Bachem AG, Bubendorf, Schweiz mit der CAS-Nummer 501-36-0 eingesetzt. Die CAS-Nummer ist ein internationaler Bezeichnungsstandard für chemische Stoffe. Für jeden bekannten chemischen Stoff existiert eine eindeutige CAS-Nummer.

Mittelkettige Triglyceride wurden als MCT-Öl (70/30) Rofetan GTCC 70/30 des Herstellers DHW Deutsche Hydrierwerke Rodleben GmbH, Dessau-Roßlau, Deutschland, CAS-Nummer 73-398-61-5 eingesetzt.

Als Polysorbat 80 (E433, CAS-Nummer 9005-65-6) sind handelsübliche Präparate wie beispielsweise TEGO SMO 80 V, Evonik oder Crillet 4/Tween 80-LQ-(SG), Croda GmbH, Nettetal, Deutschland verwendbar.

Als Polysorbat 20 (E432, CAS-Nummer 9005-64-5) sind handelsübliche Präparate wie beispielsweise TEGO SML 20 V, Evonik oder Crillet 1/Tween 20-LQ-(SG), Croda GmbH, Nettetal, Deutschland verwendbar.

Als Mischtocopherol (E306, CAS-Nummern 59-02-9, 16698-35-4, 54-28-4 und 119-13-1) kann beispielsweise das 70%ige Mischtocopherol in Pflanzenöl Vitapherole T-70 Non GMO des Herstellers Vitae Caps S.A., Spanien, oder EMix 70 der Nutrilo GmbH, Cuxhaven, Deutschland eingesetzt werden.

Polysorbat 20, Polysorbat 80, Mischtocopherol und MCT-Öl werden bei einer Temperatur im Bereich zwischen etwa 18°C und etwa 22°C unter Rühren homogenisiert.

In die Mischung aus Polysorbat 20, Polysorbat 80, Mischtocopherol und MCT-Öl wird Resveratrol gegeben und zum Homogenisieren unter Rühren erhitzt auf eine Temperatur im Bereich zwischen etwa 83°C und etwa 87°C. Sobald das Fluid homogen und transparentes ist, wird es auf eine Temperatur unterhalb von etwa 30°C abgekühlt.

Das entstehende Solubilisat ist ein hellbraunes viskoses Fluid, das im Verhältnis 1:50 mit Wasser verdünnt eine leicht gelbliche klare Lösung ergibt. Der Resveratrol-Gehalt des Solubilisats beträgt nach einer HPLC-Analyse mindestens 10 Gew.-%, wobei das Resveratrol in Micellen eingeschlossen ist. Die Dichte des Solubilisats liegt nach einer Aräometermessung im Bereich zwischen 1,05 und 1,15 g/cm$^3$ bei einer Temperatur von 20°C. Die Trübung des Solubilisats liegt bei kleiner oder gleich 50 FNU bei einer Lösung in Wasser im Verhältnis 1:50. Diese Lösung hat einen pH-Wert im Bereich zwischen 6 und 8 gemäß potentiometrischer Bestimmung.

**Patentansprüche**

1. Solubilisat bestehend aus
   Resveratrol und
   Polysorbat 80 und Polysorbat 20
   sowie zumindest einem mittelkettigen Triglycerid (MCT) und optional als weiterem Bestandteil Tocopherol, insbesondere Mischtocopherol,
   und/oder optional dem weiteren Bestandteil zumindest eines anderen Polyphenols, insbesondere Quercetin und/oder Catechin.

2. Solubilisat nach Anspruch 1,
   **dadurch gekennzeichnet, dass**
   der Anteil an Resveratrol im Bereich zwischen 3 Gew.-% und 15 Gew.-% liegt, besonders bevorzugt im Bereich zwischen 5 Gew.-% und 10 Gew.-%, und insbesondere **dadurch gekennzeichnet, dass** der Anteil an Resveratrol 10 Gew.-% beträgt.

3. Solubilisat nach Anspruch 1 oder 2,
   **dadurch gekennzeichnet, dass**
   die Emulgatormischung aus Polysorbat 20 und Polysorbat 80 in einer Menge im Bereich zwischen mindestens etwa 65 Gew.-% und etwa 95 Gew-% vorliegt, insbesondere in einer Menge im Bereich zwischen etwa 70 Gew.-% und etwa 92 Gew.-%, wobei der Anteil der Emulgatormischung aus Polysorbat 20 und Polysorbat 80 bevorzugt etwa 71,8 Gew.-% beträgt.

4. Solubilisat nach einem der vorangegangenen Ansprüche,
   **dadurch gekennzeichnet, dass**
   das zumindest eine mittelkettiges Triglycerid in einer Menge im Bereich zwischen mindestens etwa 2 Gew.-% und etwa 8 Gew-% vorliegt, insbesondere in einer Menge im Bereich zwischen etwa 3 Gew.-% und etwa 5 Gew.-%, wobei der Anteil des zumindest einen mittelkettigen Triglycerids (MCT-Anteil) bevorzugt etwa 4,5 Gew.-% beträgt.

5. Solubilisat nach einem der vorangegangenen Ansprüche, dadurch gekennzeichnet, dass
   der Tocopherolanteil liegt im erfindungsgemäßen Solubilisat in einer Menge im Bereich bis etwa 10 Gew.-%, insbesondere in einer Menge im Bereich zwischen etwa 3 Gew.-% und etwa 6 Gew.-% vor, und bevorzugt beträgt der Tocopherolanteil etwa 5,25 Gew.-%.

6. Solubilisat nach einem der vorangegangenen Ansprüche, dadurch gekennzeichnet, dass
   die Durchmesserverteilung der Micellen von etwa 1 nm bis etwa 25 nm reicht.

7. Solubilisat nach einem der vorangegangenen Ansprüche, dadurch gekennzeichnet, dass
   die Trübung des Solubilisats kleiner als 50 FNU gemessen durch Streulichtmessung mit Infrarotlicht nach den Vorschriften der Norm ISO 7027 bei einer Verdünnung des Solubilisats im Verhältnis 1:50 in Wasser.

8. Solubilisat nach einem der vorangegangenen Ansprüche,
   **dadurch gekennzeichnet, dass**
   die Trübung des Solubilisats nach 24 Stunden Lagerung bei Raumtemperatur und pH 7 kleiner als 50 FNU ist gemessen durch Streulichtmessung mit Infrarotlicht nach den Vorschriften der Norm ISO 7027 bei einer Verdünnung des Solubilisats im Verhältnis 1:50 in Wasser.

9. Solubilisat nach einem der vorangegangenen Ansprüche, dadurch gekennzeichnet, dass
   die Trübung des Solubilisats nach 1 Stunde Lagerung bei 37°C und pH 1,1 kleiner als 50 FNU ist gemessen durch Streulichtmessung mit Infrarotlicht nach den Vorschriften der Norm ISO 7027 bei einer Verdünnung des Solubilisats im Verhältnis 1:50 in Wasser.

10. Kapsel gefüllt mit einem Solubilisat nach einem der vorangegangenen Ansprüche,
    **dadurch gekennzeichnet, dass**
    die Kapsel als Weichgelatinekapsel oder Hartgelatinekapsel oder als weiche, gelatinefreie Kapsel oder als harte, gelatinefreie Kapsel ausgebildet ist.

11. Verwendung eines Solubilisats nach einem der Ansprüche 1 bis 9, insbesondere in wässriger Verdünnung, in einem Lebensmittel.

**12.** Verwendung eines Solubilisats nach einem der Ansprüche 1 bis 9, insbesondere in wässriger Verdünnung, in einem Getränk.

**13.** Verwendung eines Solubilisats nach einem der Ansprüche 1 bis 9, insbesondere in wässriger Verdünnung, in einem kosmetischen Produkt, insbesondere einer Creme, einer Lotion oder einer Salbe.

**Claims**

**1.** Solubilisation product consisting of
resveratrol and
polysorbate 80 and polysorbate 20
as well as at least one medium-chain triglyceride (MCT) and optionally tocopherol, in particular mixed tocopherol, as a further component,
and/or optionally as a further component, a further polyphenol, in particular quercetin and/or catechin.

**2.** Solubilisation product according to claim 1, characterised in that
the resveratrol fraction is in the range of 3% by weight to 15% by weight, particularly preferably in the range of 5% by weight to 10% by weight, and in particular is **characterised in that** the resveratrol fraction is 10% by weight.

**3.** Solubilisation product according to claim 1 or 2, characterised in that
the amount of the emulsifying agent mixture made up of polysorbate 20 and polysorbate 80 is in the range of approximately 65% by weight to approximately 95% by weight, in particular in the range of approximately 70% by weight to approximately 92% by weight, whereby the fraction of the emulsifying agent mixture made up of polysorbate 20 and polysorbate 80 preferably is approximately 71.8% by weight.

**4.** Solubilisation product according to any one of the preceding claims,
**characterised in that**
the amount of the at least one medium-chain triglyceride is in the range of at least approximately 2% by weight to approximately 8% by weight, in particular in the range of approximately 3% by weight to approximately 5% by weight, whereby the fraction of the at least one medium-chain triglyceride (MCT fraction) preferably is approximately 4.5% by weight.

**5.** Solubilisation product according to any one of the preceding claims,
**characterised in that**
the tocopherol content in the solubilisation product according to the invention is in the range of approximately 3% by weight to approximately 6% by weight,
and preferably the tocopherol fraction is approximately 5.25% by weight.

**6.** Solubilisation product according to any one of the preceding claims,
**characterised in that**
the distribution of the diameters of the micelles ranges from approximately 1 nm to approximately 25 nm.

**7.** Solubilisation product according to any one of the preceding claims,
**characterised in that**
the turbidity of the solubilisation product is less than 50 FNU, measured by scattered light measurement with infrared light according to the provisions of the ISO 7027 standard at a 1:50 dilution of the solubilisation product in water.

**8.** Solubilisation product according to any one of the preceding claims,
**characterised in that**
the turbidity of the solubilisation product after 24 hours of storage at room temperature and pH 7 is less than 50 FNU, measured by scattered light measurement with infrared light according to the provisions of the ISO 7027 standard at a 1:50 dilution of the solubilisation product in water.

**9.** Solubilisation product according to any one of the preceding claims,
**characterised in that**
the turbidity of the solubilisation product after 1 hour of storage at 37°C and pH 1.1 is less than 50 FNU, measured by scattered light measurement with infrared light according to the provisions of the ISO 7027 standard at a 1:50 dilution of the solubilisation product in water.

**10.** Capsule filled with a solubilisation product according to any one of the preceding claims,
**characterised in that**
the capsule is provided as soft gelatine capsule or hard gelatine capsule or as soft gelatin-free capsule or as hard gelatine-free capsule.

**11.** Use of a solubilisation product according to any one of claims 1 to 9, in particular in aqueous dilution, in a food.

**12.** Use of a solubilisation product according to any one of claims 1 to 9, in particular in aqueous dilution, in a beverage.

**13.** Use of a solubilisation product according to any one of claims 1 to 9, in particular in aqueous dilution, in a cosmetic product, in particular a cream, a lotion or an ointment.

**Revendications**

1. Solubilisat composé de
resvératrol et de
polysorbate 80 et polysorbate 20
ainsi qu'au moins d'un triglycéride à chaîne moyenne
(MCT) et, facultativement comme autre composé,
de tocophérol, notamment de tocophérol mélangé,
et/ou facultativement à l'autre composé, au moins
d'un autre polyphénol, notamment la quercétine
et/ou la catéchine.

2. Solubilisat conformément à la revendication 1,
**caractérisé en ce que**
le taux de resvératrol est compris entre 3% en poids
et 15% en poids, en particulier de préférence entre
5% en poids et 10% en poids, et
en particulier **caractérisé en ce que** le taux de resvératrol est de 10% en poids.

3. Solubilisat conformément à la revendication 1 ou 2,
**caractérisé en ce que**
le mélange émulsifiant composé de polysorbate 20
et polysorbate 80 est présent dans une quantité comprise entre au moins environ 65% en poids et environ
95% en poids, en particulier dans une quantité comprise entre au moins environ 70% en poids et environ
92% en poids, le taux de mélange émulsifiant composé de polysorbate 20 et polysorbate 80 étant de
préférence d'environ 71,8% en poids.

4. Solubilisat conformément à l'une des revendications
précédentes,
**caractérisé en ce que**
au moins un triglycéride à chaîne moyenne est présent dans une quantité comprise entre au moins environ 2% en poids et environ 8% en poids, en particulier dans une quantité comprise entre environ 3%
en poids et environ 5% en poids, le taux au moins
d'un triglycéride à chaîne moyenne (taux de MCT)
étant de préférence d'environ 4,5% en poids.

5. Solubilisat conformément à l'une des revendications
précédentes,
**caractérisé en ce que**
le taux de tocophérol est présent, dans le solubilisat
conforme à l'invention, dans une quantité pouvant
atteindre environ 10% en poids, en particulier dans
une quantité comprise entre environ 3% en poids et
environ 6% en poids, et le taux de tocophérol est de
préférence d'environ 5,25% en poids.

6. Solubilisat conformément à l'une des revendications
précédentes,
**caractérisé en ce que**
la répartition des diamètres des micelles s'étend
d'environ 1 nm à environ 25 nm.

7. Solubilisat conformément à l'une des revendications
précédentes,
**caractérisé en ce que**
la turbidité du solubilisat est inférieure à 50 FNU, la
mesure étant réalisée sur la lumière diffusée avec
lumière infrarouge conformément aux prescriptions
de la norme ISO 7027 avec une dilution du solubilisat
dans l'eau dans un rapport de 1:50.

8. Solubilisat conformément à l'une des revendications
précédentes,
**caractérisé en ce que**
la turbidité du solubilisat est inférieure à 50 FNU
après 24 heures de stockage à température ambiante et avec un pH de 7, la mesure étant réalisée sur
la lumière diffusée avec lumière infrarouge conformément aux prescriptions de la norme ISO 7027
avec une dilution du solubilisat dans l'eau dans un
rapport de 1:50.

9. Solubilisat conformément à l'une des revendications
précédentes,
**caractérisé en ce que**
la turbidité du solubilisat est inférieure à 50 FNU
après 1 heure de stockage à une température de
37°C et avec un pH de 1,1, la mesure étant réalisée
sur la lumière diffusée avec lumière infrarouge conformément aux prescriptions de la norme ISO 7027
avec une dilution du solubilisat dans l'eau dans un
rapport de 1:50.

10. Capsule remplie avec un solubilisat conformément
à l'une des revendications précédentes,
**caractérisée en ce que**
la capsule est conçue comme une capsule en gélatine molle ou une capsule en gélatine dure ou comme
une capsule molle, sans gélatine ou comme une capsule dure, sans gélatine.

11. Utilisation d'un solubilisat conformément à l'une des
revendications 1 à 9, en particulier en dilution aqueuse, dans un aliment.

12. Utilisation d'un solubilisat conformément à l'une des
revendications 1 à 9, en particulier en dilution aqueuse, dans une boisson.

13. Utilisation d'un solubilisat conformément à l'une des
revendications 1 à 9, en particulier en dilution aqueuse, dans un produit cosmétique, en particulier une
crème, une lotion ou une pommade.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- *Pharmazeutische Zeitung online*

- **NATHAN GRAY.** *Resveratrol could enhance exercise performance,* 20. Juni 2012, www.nutraingredients.com/content/view/print/648155